# EUROPÄISCHE PATENTSCHRIFT

(11) **EP 1 852 105 B1**
(45) Veröffentlichungstag und Bekanntmachung des Hinweises auf die Patenterteilung: **05.08.2015**
(21) Anmeldenummer: 07008739.0
(22) Anmeldetag: 28.04.2007
(51) Int. Cl.: A61K 47/36, A61K 47/40, A61K 36/185, A61K 9/00

(54) **Verfahren zur Herstellung einer triterpensäurehaltigen wässrigen Lösung, triterpensäurehaltige wässrige Lösung und deren Verwendung**
Method for manufacturing an aqueous solution containing triterpene acid, an aqueous solution containing triterpene acid and its application
Procédé de fabrication d'une solution liquide contenant des acides de titerpène, solution liquide contenant du titerpène et son utilisation

(30) Priorität: 03.05.2006 DE 102006020582
(43) Veröffentlichungstag der Anmeldung: 07.11.2007
(73) Patentinhaber: Birken AG, 75223 Niefern-Öschelbronn (DE)
(72) Erfinder: Scheffler, Armin, 75223 Niefern (DE); Jäger, Sebastian, 75446 Wiernsheim-Pinache (DE); Beffert, Markus, 75180 Pforzheim (DE); Hoppe, Katharina, 75236 Kämpelbach (DE)
(74) Vertreter: Westphal, Mussgnug & Partner Patentanwälte mbB

(56) Entgegenhaltungen:
- WO-A1-01/72315
- US-B1- 6 482 857
- DATABASE WPI Week 200540 Derwent Publications Ltd., London, GB; AN 2005-386993 XP002452192 & CN 1 579 404 A (SUN M) 16. Februar 2005 (2005-02-16)

## Beschreibung

Die vorliegende Erfindung betrifft ein Verfahren zur Herstellung einer triterpensäurehaltigen wässrigen Lösung, eine triterpensäurehaltige wässrige Lösung und deren Verwendung.

Triterpensäuren sind pharmakologisch hocheffiziente Wirkstoffe, die hauptsächlich pflanzlichen Ursprungs und in der Natur weit verbreitet sind. Triterpensäuren treten überwiegend als pentazyklische Verbindungen auf, die sich formal vom Isopren ableiten.

Die Rinden von Birken und Platanen enthalten beispielsweise Betulinsäure als pentazyklisches Triterpenoid, dessen antitumorale Aktivität in zahlreichen Veröffentlichungen beschrieben ist.

Ein Verfahren zur Extraktion von Triterpenoiden aus Pflanzen oder Pflanzenbestandteilen sind beispielsweise in der WO 2001/72315 A1 oder der WO 2004/016336 A1 beschrieben.

Die DE 100 15 353 A1 beschreibt eine ein Pflanzenextrakt enthaltene Emulsion, wobei der Pflanzenextrakt mindestens ein Triterpenoid und/oder ein Derivat eines Triterpenoids enthält. Derartige Emulsionen, die Triterpenoide in einer Konzentration von 3 bis 15% enthalten, werden dadurch erhalten, dass das bei einer Extraktion gewonnene Triterpenoid in einem Öl oder Fett dispergiert wird. Verwendung findet die Emulsion als Bestandteil einer Salbe, einer Lotion oder einer Creme zum Auftragen auf die Haut, insbesondere bei dermatologischen Veränderungen der Haut.

Ursolsäure, ebenfalls eine pentazyklische Triterpensäure, kann beispielsweise aus Apfelkernen, aber auch aus Heidel- oder Preiselbeeren gewonnen werden und ist bekannt dafür, dass sie den Stoffwechsel der Haut anregt. Glycyrrhetinsäure sowie deren Glycosid Glycyrrhizinsäure, die aus einem Extrakt aus Süßholz (Glycyrrhiza alba) gewonnen werden können, zeigen entzündungshemmende und anticancerogene Wirkungen. Boswellinsäure, die 50 % des Harzes des Weihrauchbaumes (Olibanum) ausmacht, wird als Therapeutikum in der Rheumabehandlung eingesetzt. Darüber hinaus ist Boswellinsäure bekannt für seine schmerzstillende und beruhigende Wirkung. Die ebenfalls pentazyklische Triterpensäure Oleanolsäure ist in den Blättern des Olivenbaums oder Efeus zu finden.

Oleanolsäure ist neben Betulinsäure und Ursolsäure das Haupttriterpenoid in Misteln (Viscum album L.). Ebenso wie die Betulinsäure zeigen auch Oleanolsäure und Ursolsäure antitumorale Wirkung.

Corosolsäure, die als Extrakt aus den Blättern von Lagerstroemia speciosa, dem Banaba-Baum, gewonnen werden kann, zeigt bei der oralen Verabreichung eine Glukosespiegelsenkende Wirkung.

Neben seiner antitumoralen Wirkung zeigt die in der Birkenborke, Platane oder Mistel enthaltene Triterpensäure, Betulinsäure antiseptische Wirkung sowie eine antivirale Wirkung gegen das HI-Virus.

Betulinsäure ist eine pentazyklische Triterpensäure, die zur Gruppe der Lupane gezählt wird. Das charakteristische Merkmal der Lupane ist ein Ring mit fünf Kohlenstoffatomen innerhalb eines pentazyklischen Systems. Das Gruppenmerkmal der Oleanane und Ursane ist ein pentazyklisches System nur aus Ringen mit sechs Kohlenstoffatomen, wobei sich der Ring E durch die Position einer Methylgruppe unterscheidet. Ein wichtiger Vertreter der Oleanane ist die Oleanolsäure und Ursolsäure gehört zu den Ursanen.

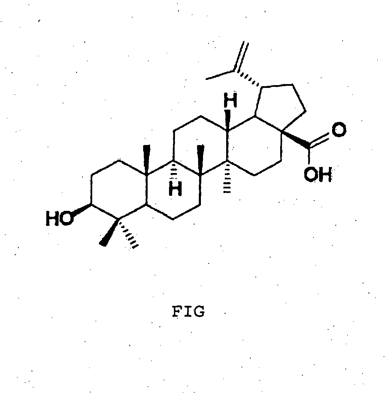

Beispielhaft für eine derartige Säure ist Betulinsäure in der beigefügten Figur wiedergegeben.

In den US-Schriften US 6,482,857 B1, US 2003/0139471 A1, US 6,451,777 B1 und US 2003/014526 A1 werden wässrige Suspensionen bzw. Emulsionen von Triterpensäuren für den Einsatz als Haartonikum beschrieben. Die WO 2006/088385 beschreibt bioaktive Komplexe von Triterpensäuren zu therapeutischen Verwendung. In der EP 1 250 852 B1 werden Ursol- und Oleanolsäurehaltige Triterpen-Konzentrate für den Einsatz in Nahrungsmitteln beschrieben.

Der Einfluss von Cyclodextrinen auf die Hydrophilie von Triterpensäuren ist einem Artikel aus dem J. Chromatogr. A, 1049 (2004), 37 - 42, ISSN: 0021 - 9673 (B. Claude et al.) sowie einem weiteren Artikel aus dem J. Mass. Spectrom. 2003; 38: 723 - 731 (Guo et al.) zu entnehmen.

Triterpensäuren zeichnen sich durch eine hohe thermische Stabilität aus. Der Schmelzpunkt von Oleanolsäure (Molekulargewicht: 456 g/mol) liegt bei über 300°C, der von Betulinsäure (Molekulargewicht: 456 g/mol) zwischen 275°C und 278°C und der von Ursolsäure ((Molekulargewicht: 456 g/mol) bei etwa 278°C.

Obwohl die pharmakologische Wirkung der Triterpenoide allgemein anerkannt ist und unerwünschte Nebenwirkungen dieser Naturstoffe bisher nicht beobachtet wurden, steht einer weiteren Anwendung dieser Wirkstoffe vor allem ihre geringe Wasserlöslichkeit entgegen. Sämtliche vorher genannten Triterpenoide oder Extrakte mit Triterpenoiden haben den Nachteil, dass die Wirkstoffe in einem wässrig physiologischen System nur unzureichend gelöst werden können, so dass eine für eine Applikation, z.B. in Form von Injektionspräparaten, ausreichende Menge und Konzentration an Wirkstoff bisher nicht erhalten werden konnte.

Triterpensäuren, wie beispielsweise Oleanolsäure, Betulinsäure oder Ursolsäure, sind löslich in Pyridin und Tetrahydrofuran, aber nur gering löslich in Dichlormethan, Chloroform und kalten organischen Lösungsmitteln, wobei sich die Löslichkeit mit steigender Temperatur erheblich verbessert. In Wasser sind sie mit weniger als 0,1 µg/mL praktisch unlöslich.

Die CN 1579404 (Derwent Publications) beschreibt eine wässrige triterpensäurehaltige Lösung.

Die US 6,482,857 B1 beschreibt eine injizierbare Lösung die Triterpene und ein Vehikel enthält, wobei das Vehikel beispielsweise Wasser, ein lipophiler oder hydrophiler Weichmacher, ein Verdickungsmittel, ein Polymer, ein Harz, usw. ist.

Die zuvor genannte WO 01/72315 A1 beschreibt außerdem eine Emulsion, die ein Triterpen und/oder ein Derivat eines Triterpens aufweist, wobei das Triterpen und/oder das Derivat die Emulsion emulgiert und stabilisiert und pharmazeutisch aktiv ist.

Aufgabe der vorliegenden Erfindung ist es daher ein Verfahren zur Herstellung einer triterpensäurehaltigen wässrigen Lösung, eine triterpensäurehaltige wässrige Lösung und ein Verfahren zur Verwendung einer solchen wässrigen Lösung zur Verfügung zu stellen.

Diese Aufgabe wird durch ein Verfahren nach Anspruch 1, eine wässrige Lösung nach Anspruch 14, sowie durch Verwendungen nach den Ansprüchen 19 bis 25 gelöst. Vorteilhafte Ausgestaltungen der Erfindung sind Gegenstand der Unteransprüche.

Das Verfahren zur Herstellung einer wässrigen triterpensäurehaltigen Lösung sieht das Bereitstellen einer basischen wässrigen triterpensäurehaltigen Lösung und das Reduzieren des pH-Wertes der basischen triterpensäurehaltigen Lösung durch Zugabe einer sauren wässrigen Lösung in Gegenwart eines stabilisierenden Additivs vor.

Unter einer triterpensäurehaltigen Lösung ist im Zusammenhang mit der vorliegenden Erfindung eine Lösung zu verstehen, die wenigstens eine Triterpensäure und/oder ein Derivat einer Triterpensäure enthält. Die wenigstens eine Triterpensäure und/oder das Derivat einer Triterpensäure umfasst beispielsweise Asiatinsäure, Betulinsäure, Boswellinsäure, Corosolsäure, Glycyrrhetinsäure, Madecassinsäure, Oleanolsäure, Phellonsäure und/oder Ursolsäure.

Unter einer wässrig-physiologischen Lösung ist nachfolgend eine wässrige Lösung mit einem pH-Wert zwischen 6 und 8 zu verstehen.

Zyklische Triterpenoide mit nicht mehr als zwei funktionellen sauerstoffreichen Gruppen sind in wässrig-physiologischen Lösungen sehr schwer löslich. So beträgt beispielsweise die Löslichkeit von Betulinsäure in Wasser weniger als 0,1 µg/ml. Die Gegenwart eines stabilisierenden Additivs ermöglicht es, bei dem erfindungsgemäßen Verfahren den pH-Wert der basischen wässrigen Lösung auf den pH-Wert einer wässrig-physiologischen Lösung zu reduzieren, ohne dass Ausfällungen der Triterpensäuren auftreten. Auf diese Weise wird eine wässrig-physiologische Lösung erhalten, in der therapeutisch relevante Mengen wenigstens einer Triterpensäure oder deren Derivats vorhanden sind.

Die Herstellung der basischen triterpensäurehaltigen Lösung kann auf herkömmliche Weise durch Lösen wenigstens einer Triterpensäure oder deren Derivats in einem geeigneten basischen Medium, wie z.B. Trinatriumphosphat in Wasser, erfolgen. Die Triterpensäure kann hierbei vor dem Lösen in Form eines hochkonzentrierten kristallinen Substrats vorliegen, das zuvor durch Extraktion aus Pflanzen oder Pflanzenbestandteilen gewonnen wurde.

Als basische triterpensäurehaltige Lösung eignet sich jedoch auch unmittelbar eine durch Extraktion von Triterpensäurenen aus Pflanzen oder deren Bestandteilen erhaltene Extraktlösung. Zur Gewinnung einer solchen Extraktlösung eignet sich ein beliebiges bekanntes Verfahren zur Extraktion von Triterpenoiden aus Pflanzen oder deren Bestandteilen unter Verwendung eines basischen Extraktionsmittels.

Das Lösen der Triterpensäuren in der basischen wässrigen Lösung kann bei Temperaturen zwischen 20°C und 200°C und Drücken zwischen 5 bar und 150 bar erfolgen.

Der pH-Wert der basischen wässrigen Lösung wird beispielsweise auf einen Wert zwischen 8 und 14 eingestellt. Geeignet zur Einstellung des pH-Werts ist beispielsweise Trinatriumphosphat.

Zur Reduzierung des pH-Wertes der basischen wässrigen triterpensäurehaltigen Lösung auf physiologische Werte (pH-Wert zwischen 6 und 8) eignen sich physiologisch verträgliche Säuren, wie z. B. Phosphorsäure oder Zitronensäure.

Als stabilisierende Additive, die eine Ausflockung bei Reduktion des pH-Werts verhindern, eignen sich insbesondere Polysaccharide.

Als pflanzliche Rohstoffe für die Gewinnung des wenigstens einer, in der wässrigen Lösung enthaltenen Triterpensäure eignen sich besonders gut Misteln, Birken oder Platanen, die jeweils hohe Anteile an Oleanolsäure bzw. Betulinsäure enthalten. Es sei angemerkt, dass triterpenoidhaltige Pflanzen üblicherweise verschiedene Triterpenoide enthalten, die in dem durch Extraktion der Pflanzen erhaltenen Extrakt in entsprechender Konzentration enthalten sind. So enthält Mistel neben Oleanolsäure und Betulinsäure in geringerer Konzentration noch Ursolsäure, β-Amyrin, Lupeol, β-Amyrinacetat und Lupeolacetat.

Je nach verwendetem Extrakt zur Herstellung der basischen triterpenoidhaltigen Lösung, d.h. je nach Pflanzenmaterial zur Gewinnung des Extrakts, sind in dieser Lösung verschiedene Triterpenoide in verschiedener Konzentration vorhanden.

Als stabilisierendes Additiv kann insbesondere ein dem jeweiligen Pflanzenmaterial entsprechendes Polysaccharid eingesetzt werden. So kann beispielsweise bei der Gewinnung eines wässrig-physiologischen Mistelextraktes zur Stabilisierung der Triterpensäuren ein polysaccharidhaltiger Extrakt aus Mistelbeeren eingesetzt werden.

Die Salzkonzentration der neutralisierten (physiologischen), triterpensäurehaltigen Lösung kann auf geeignete gewünschte Werte, beispielsweise 3 bis 140 mM, bevorzugt 10 mM, eingestellt werden. Je nach Applikationswunsch kann z.B. für eine subcutane Injektionslösung noch eine Isotonisierung z.B. auch mit Sacchariden vorgenommen werden.

Das oben erläuterte Verfahren ermöglicht es, unter Verwendung beliebiger Triterpensäuren triterpensäurehaltige injizierbare therapeutische Präparate herzustellen, deren Triterpensäuregehalt weit über der Löslichkeitsgrenze von Triterpenen in Wasser liegt und die einen Triterpenoidgehalt von mehr als 0,5µg/ml, bevorzugt mehr als 1 µg/ml, aufweisen.

Ein besonderes Potential der vorliegenden Erfindung besteht darin, dass die triterpensäurehaltigen wässrig-physiologischen Lösungen nicht nur für sich eingesetzt werden können, sondern darüber hinaus auch mit anderen wässrig-physiologischen Pflanzenextrakten, die keine Triterpenoide, aber dafür andere Wirkstoffe entsprechender Pflanzen enthalten, kombiniert werden können. Durch diese Kombination gelingt es, zusätzliche Wirkstoffe in das wässrig-physiologische Präparat einzuführen. Ein solcher anderer Extrakt ist beispielsweise ein vesikelhaltiger Mistelextrakt.

Überraschenderweise wurde bei der Kombination mit anderen Pflanzenextrakten gefunden, dass die triterpensäurehaltige Lösung, die anderen Pflanzenextrakte gegen Ausflockung stabilisiert. Da ein Großteil aller wässrigen Pflanzenextrakte zur Ausflockung neigt, ist dieser positive Effekt besonders wichtig für die Anwendung und Aufbewahrung von therapeutischen Pflanzenextrakten. Ein entsprechend flockungsstabilisierter Extrakt enthält nun neben weiteren pflanzlichen Wirkstoffen darüber hinaus eine therapeutisch wirksame Menge an Triterpensäuren.

Die Kombination einer triterpensäurehaltigen wässrig-physiologischen Lösung mit einem weiteren Pflanzenextrakt, der insbesondere ein wässriger Pflanzenextrakt einer triterpenoidhaltigen Pflanze oder von Teilen davon sein kann, und die dabei beobachtete Stabilisierung der wässrig-physiologischen, mehrere Pflanzenextrakte enthaltenen Lösung gegen eine Ausflockung eröffnet ungeahnte therapeutische Möglichkeiten für den Einsatz von Pflanzenextrakten. So können auf diese Weise triterpensäurehaltige injizierbare Präparate formuliert werden, denen neben den Triterpensäüren gezielt weintere pflanzliche Wirkstoffe zugesetzt werden. Diese zusätzliche pflanzlichen Wirkstoffe beschränken sich dabei nicht auf Triterpenoide, sondern es sind sämtliche Wirkstoffe denkbar, die als Pflanzenextrakt gewonnen werden können.

Die flockungsstabilisierende Wirkung der triterpensäurereichen Lösung basiert nach ersten Untersuchungen auf einer Kombination der Triterpensäuren mit dem wenigstens einen Stabilisator.

Die vorliegende Erfindung wird nachfolgend anhand von Beispielen erläutert:

### Beispiel 1

Getrocknete, einjährige Triebe der Mistel werden mit n-Heptan bei 120°C und > 2 bar Druck extrahiert. Der beim Abkühlen des Extraktionsmittels erhaltene Niederschlag besteht zu mehr als 60 % aus Oleanolsäure und zu mehr als 3 % aus Betulinsäure. Das so gewonnene triterpensäurereiche Pulver wird mit Wasser in Gegenwart von Trinatriumphosphat bei 120°C und > 2 bar Druck zu einer übersättigten basischen Lösung extrahiert.

Die basische, auf Raumtemperatur abgekühlte und durch 0,22 µm filtrierte triterpenoidreiche Lösung enthält mehr als 80 µg/ml Oleanolsäure und mehr als 8 µg/ml Betulinsäure. Die so hergestellte basische Lösung wird anschließend in Gegenwart von 1,6 µg/ml Polysacchariden, die aus Mistelbeeren gewonnen wurden, mit einer wässrigen Phosphorsäurelösung auf einen pH-Wert von 7,4 eingestellt. Der Verdünnungsgrad und die Lösungsmittelmengen werden dabei so gewählt, dass die Salzkonzentration der neutralisierten triterpensäurereichen Lösung zwischen 5 und 140 mM liegt.

### Beispiel 2

Ein Teil eines wässrig-physiologischen wirkstoffreichen, aber triterpenoidarmen Mistelextraktes wird mit einem Teil einer wässrig-physiologischen Lösung eines Mistelextraktes aus Beispiel 1 vermischt.

Man erhält auf diese Weise einen wässrig-physiologischen Pflanzenextrakt, der flockungsstabilisiert ist und neben den sonstigen, wässrig-extrahierbaren Wirkstoffen der Mistel 90% der aus dem basischen Extrakt stammenden Triterpensäuren enthält.

### Beispiel 3

Zwei Teile wässrig-physiologischer, triterpenoidarmer Mistelextrakt werden mit jeweils einem Teil basischer Triterpensäurelösung und einem Teil saurer Polysaccharidlösung versetzt.

Auf diese Weise erhält man, wie in Beispiel 2, eine wässrig-physiologische Lösung, die neben den wässrig-extrahierbaren Wirkstoffen der Mistel therapeutisch wirksame Mengen an Triterpensäuren enthält. Der so hergestellte Pflanzenextrakt ist gegen Ausflockung stabilisiert.

### Beispiel 4

Das im Beispiel 1 beschriebene triterpenoidreiche Pulver wird mit Wasser in Gegenwart von Trinatriumphosphat (10 mM) und 2 mg/mL Cyclodextrinen bei 120 °C und > 2 bar extrahiert.

Die basische triterpensäurereiche Lösung enthält mehr als 100 µg/mL Oleanolsäure und mehr als 8 µg/mL Betulinsäure. Die So hergestellte basische Lösung wird anschließend mit einer wässrigen Phosphorsäurelösung auf einen pH Wert von 7,4 eingestellt. Der Verdünnungsgrad und die Lösungsmittelmengen werden dabei so gewählt, dass die Salzkonzentration der neutralisierten triterpensäurereichen Lösung zwischen 5 und 140 mM liegt.

Diese wässrig- pyhsiologische und triterpensäurereiche Lösung kann so eingesetzt werden, wie es das Beispiel 2 beschreibt.

Weiterhin kann die basische, cyclodextrine und triterpensäuren enthaltende Lösung dieses Beispieles wie im Beispiel 3 beschrieben verwendet werden.

### Beispiel 5

Während der im Beispiel 4 beschriebenen Neutralisation können Polysaccharide, die aus Mistelbeeren gewonnen wurden, anwesend sein.

### Beispiel 6

Für das im Beispiel 3 beschriebene Verfahren kann die im Beispiel 4 beschriebene, cyclodextrinhaltige und triterpensäurereiche Lösung verwendet werden. Die erzielten Effekte gleichen dabei denen, die im Beispiel 3 beschrieben sind.

## Patentansprüche

1. Verfahren zur Herstellung einer wässrigen Lösung mit den Verfahrensschritten:
a) Bereitstellen einer basischen wässrigen eine Triterpensäure und/oder ein Derivat einer Triterpensäure enthaltenden Lösung,
b) Neutralisieren des pH-Wertes der basischen wässrigen eine Triterpensäure und/oder ein Derivat einer Triterpensäure enthaltenden Lösung durch Zugabe einer sauren wässrigen Lösung in Gegenwart eines Polysaccharids oder Oligosaccharids als stabilisierendes Additiv,
wobei der basischen wässrigen Lösung in Schritt a) oder der pH-Wert reduzierten Lösung nach Schritt b) oder während der Durchführung von Schritt b) wenigstens ein weiterer wässriger Pflanzenextrakt zugegeben wird, der ein Pflanzenextrakt einer triterpenoidhaltigen Pflanze oder von Teilen einer triterpenoidhaltigen Pflanze ist, und
wobei das Derivat einer Triterpensäure das Derivat von Asiatinsäure, Betulinsäure, Boswellinsäure, Corosolsäure, Glycyrrhetinsäure, Madecassinsäure, Oleanolsäure, Phellonsäure und/oder Ursolsäure umfasst.

2. Verfahren nach Anspruch 1, bei dem das Oligosaccharid ein Cyclodextrin ist.

3. Verfahren nach Anspruch 1 oder 2, bei dem der pH-Wert der basischen wässrigen Lösung in Schritta) zwischen 8 und 14 beträgt.

4. Verfahren nach einem der vorangehenden Ansprüche, bei dem der pH-Wert der Lösung in Schritt b) bis auf einen Wert zwischen 6 und 8 reduziert wird.

5. Verfahren nach einem der vorangehenden Ansprüche, bei dem das Bereitstellen der basischen wässrigen Lösung den Verfahrensschritt umfasst:
a1) Bereitstellen eines triterpenoidhaltigen Feststoffes,
a2) Lösen des triterpenoidhaltigen Feststoffes in einem basischen wässrigen Medium bei einer Temperatur zwischen 20 °C und 200 °C.

6. Verfahren nach Anspruch 5, bei dem der Druck in Schritt a2) zwischen 2 und 150 bar beträgt.

7. Verfahren nach einem der vorangehenden Ansprüche, bei dem zur Einstellung des pH-Wertes in Schritt a) Trinatriumphosphat eingesetzt wird.

8. Verfahren nach einem der vorangehenden Ansprüche, bei dem zur Reduzierung des pH-Wertes in Schritt b) Phosphorsäure und/oder Zitronensäure eingesetzt wird.

9. Verfahren nach einem der vorangehenden bei dem das stabilisierende Additiv der sauren wässrigen Lösung zugegeben ist.

10. Verfahren nach einem der Ansprüche 1 bis 9 bei dem das stabilisierende Additiv der basischen wässrigen Lösung zugegeben ist.

11. Verfahren nach einem der vorangehenden Ansprüche, bei dem der wenigstens eine weitere wässrige Pflanzenextrakt ein Pflanzenextrakt aus Misteln, Birken und/oder Platanen oder Teilen davon ist.

12. Verfahren nach einem der vorangehenden Ansprüche, bei dem der wenigstens eine weitere Pflanzenextrakt einen pH-Wert zwischen 5 und 9 aufweist.

13. Verfahren nach einem der vorangehenden Ansprüche, bei dem der wenigstens eine weitere Extrakt ein vesikelhaltiger Pflanzenextrakt ist.

14. Wässrige Lösung eines Pflanzenextraktes, der wenigstens eine Triterpensäure und/oder ein Derivat einer Triterpensäure aufweist, wobei die Lösung bei einem pH-Wert von 5 bis 9 in Gegenwart eines die wässrig-physiologische Lösung von Triterpensäuren stabilisierenden Additivs einen Triterpensäuregehalt von mehr als 1 µg/ml aufweist, wobei das stabilisierende Additiv ein Polysaccharid oder ein Cyclodextrin ist und bei der das Derivat einer Triterpensäure Asiatinsäure, Betulinsäure, Boswellinsäure, Corosolsäure, Glycyrrhetinsäure, Madecassinsäure, Oleanolsäure, Phellonsäure und/oder Ursolsäure umfasst.

15. Lösung nach Anspruch 14, bei der der Pflanzenextrakt ein Extrakt aus Misteln, aus Birken, aus Platanen und/ oder anderen triterpenoidhaltigen Pflanzen oder Teilen davon ist.

16. Lösung nach einem der Ansprüche 17 bis 19, bei der die Salzkonzentration in der Lösung 5 - 140 mM beträgt.

17. Lösung nach Anspruch 16, deren Salzkonzentration zwischen 25mM und 35mM beträgt.

18. Lösung nach einem der Ansprüche 14 bis 17, die einen vesikelhaltigen Mistelextrakt enthält.

19. Verwendung einer wässrigen Lösung nach einem der Ansprüche 14 bis 17 zur Herstellung eines Therapeutikums.

20. Verwendung einer wässrigen Lösung nach einem der Ansprüche 14 bis 17 zur Herstellung eines Nahrungsergänzungsmittels.

21. Verwendung einer wässrigen Lösung nach einem der Ansprüche 14 bis 17 zur Herstellung eines physiologisch injizierbaren Präparats.

22. Verwendung einer wässrigen Lösung nach einem der Ansprüche 14 bis 17 zur Herstellung eines physiologisch injizierbaren Präparats für die Tumortherapie.

23. Verwendung einer wässrigen Lösung nach einem der Ansprüche 14 bis 17 zur Herstellung eines physiologisch injizierbaren Präparats für die Hepatitistherapie.

24. Verwendung einer wässrigen Lösung nach einem der Ansprüche 14 bis 17 für die Herstellung eines Therapeutikums zur therapeutischen Behandlung von Tumorerkrankungen der Haut.

25. Verwendung einer wässrigen Lösung nach einem der Ansprüche 14 bis 17 zur Herstellung eines Therapeutikums für die Behandlung gegen Viren und Bakterien.

## Claims

1. Method of preparing an aqueous solution with the method steps:
a) providing a basic aqueous solution comprising a triterpene acid and/or triterpene acid derivative,
b) neutralizing the pH of the basic aqueous solution comprising a triterpene acid and/or a triterpene acid derivative by adding an acidic aqueous solution in the presence of a polysaccharide or oligosaccharide as stabilizing additive,
where at least one further aqueous plant extract is added to the basic aqueous solution in step a) or to the pH-reducing solution according to step b) or while carrying out step b), which further aqueous plant extract is a plant extract from a triterpenoid-containing plant or of parts of a triterpenoid-containing plant, and
where the derivative of a triterpene acid comprises the derivative of asiatic acid, betulinic acid, boswellic acid, corosonic acid, glycyrrhetinic acid, madecassic acid, oleanolic acid, phellonic acid and/or ursolic acid.

2. Method according to Claim 1, in which the oligosaccharide is a cyclodextrin.

3. Method according to Claim 1 or 2, in which the pH of the basic aqueous solution in step a) is between 8 and 14.

4. Method according to one of the preceding claims, in which the pH of the solution in step b) is reduced down to a value of between 6 and 8.

5. Method according to one of the preceding claims, in which providing the basic aqueous solution comprises the method step:
a1) providing a triterpenoid-containing solid,
a2) dissolving the triterpenoid-containing solid in a basic aqueous medium at a temperature of between 20°C and 200°C.

6. Method according to Claim 5, in which the pressure in step a2) is between 2 and 150 bar.

7. Method according to one of the preceding claims, in which trisodium phosphate is employed for adjusting the pH in step a).

8. Method according to one of the preceding claims, in which phosphoric acid and/or citric acid is employed for reducing the pH in step b).

9. Method according to one of the preceding claims, in which the stabilizing additive is added to the acidic aqueous solution.

10. Method according to one of Claims 1 to 9, in which the stabilizing additive is added to the basic aqueous solution.

11. Method according to one of the preceding claims, in which the at least one further aqueous plant extract is a plant extract from mistletoe, birches and/or planes or parts of these.

12. Method according to one of the preceding claims, in which the at least one further plant extract has a pH of between 5 and 9.

13. Method according to one of the preceding claims, in which the at least one further extract is a vesicle-containing plant extract.

14. Aqueous solution of a plant extract which includes at least one triterpene acid and/or triterpene acid derivative, wherein the solution has a triterpene acid content of more than 1 µg/ml at a pH of from 5 to 9 in the presence of an additive stabilizing the aqueous-physiological solution of triterpene acids, wherein the stabilizing additive is a polysaccharide or a cyclodextrin and in which the derivative of a triterpene acid comprises asiatic acid, betulinic acid, boswellic acid, corosonic acid, glycyrrhetinic acid, madecassic acid, oleanolic acid, phellonic acid and/or ursolic acid.

15. Solution according to Claim 14, in which the plant extract is an extract from mistletoe, from birches, from planes and/or from other triterpenoid-containing plants or parts thereof.

16. Solution according to one of Claims 17 to 19, in which the salt concentration in the solution amounts to 5 - 140 mM.

17. Solution according to Claim 16, whose salt concentration amounts to between 25 mM and 35 mM.

18. Solution according to one of Claims 14 to 17, which comprises a vesicle-containing mistletoe extract.

19. Use of an aqueous solution according to one of Claims 14 to 17 for the preparation of a therapeutic.

20. Use of an aqueous solution according to one of Claims 14 to 17 for the preparation of a food supplement.

21. Use of an aqueous solution according to one of Claims 14 to 17 for the preparation of a physiologically injectable preparation.

22. Use of an aqueous solution according to one of Claims 14 to 17 for the preparation of a physiologically injectable preparation for tumour therapy.

23. Use of an aqueous solution according to one of Claims 14 to 17 for the preparation of a physiologically injectable preparation for hepatitis therapy.

24. Use of an aqueous solution according to one of Claims 14 to 17 for the preparation of a therapeutic for therapeutic treatment of tumour diseases of skin.

25. Use of an aqueous solution according to one of Claims 14 to 17 for the preparation of a therapeutic for the treatment against viruses and bacteria.

## Revendications

1. Procédé de préparation d'une solution aqueuse ayant les stades de procédé suivants :
a) on se procure une solution aqueuse basique contenant un acide triterpénique et/ou un dérivé d'un acide triterpénique,
b) on rend neutre le pH de la solution aqueuse basique contenant un acide triterpénique et/ou un dérivé d'un acide triterpénique par addition d'une solution aqueuse acide en présence d'un polysaccharide ou d'un oligosaccharide comme additif stabilisant,
dans lequel on ajoute à la solution aqueuse basique au stade a) ou à la solution d'abaissement du pH suivant le stade b) ou pendant que l'on effectue le stade b) au moins un autre extrait végétal aqueux, qui est un extrait végétal d'une plante contenant un triterpénoïde ou des parties d'une plante contenant un triterpénoïde et
dans lequel le dérivé d'un acide triterpénique comprend le dérivé de l'acide asiatique, de l'acide bétulique, de l'acide boswellique, de l'acide corrosolique, de l'acide glycyrrhétinique, de l'acide madécassique, de l'acide oléanolique, de l'acide phellonique et/ou de l'acide ursolique.

2. Procédé suivant la revendication 1, dans lequel l'oligosaccharide est une cyclodextrine.

3. Procédé suivant la revendication 1 ou 2, dans lequel le pH de la solution aqueuse basique au stade a) est compris entre 8 et 14.

4. Procédé suivant l'une des revendications précédentes, dans lequel on abaisse le pH de la solution au stade b) jusqu'à une valeur comprise entre 6 et 8.

5. Procédé suivant l'une des revendications précédentes, dans lequel préparer la solution aqueuse basique comprend le stade de procédé :
a1) on se procure une substance solide contenant un triterpénoïde,
a2) on dissout la substance solide contenant un triterpénoïde dans un milieu aqueux basique à une température comprise entre 20°C et 200°C.

6. Procédé suivant la revendication 5, dans lequel la pression au stade a2) est comprise entre 2 et 150 bar.

7. Procédé suivant l'une des revendications précédentes, dans lequel on utilise du phosphate trisodique pour régler le pH au stade a).

8. Procédé suivant l'une des revendications précédentes, dans lequel on utilise de l'acide phosphorique et/ou de l'acide citrique pour diminuer le pH au stade b).

9. Procédé suivant l'une des revendications précédentes, dans lequel l'additif stabilisant est ajouté à la solution aqueuse acide.

10. Procédé suivant l'une des revendications 1 à 9, dans lequel l'additif stabilisant est ajouté à la solution aqueuse basique.

11. Procédé suivant l'une des revendications précédentes, dans lequel le au moins un autre extrait végétal aqueux est un extrait de plante de gui, de bouleau et/ou de platane ou de parties de ceux-ci.

12. Procédé suivant l'une des revendications précédentes, dans lequel le au moins un autre extrait végétal a un pH compris entre 5 et 9.

13. Procédé suivant l'une des revendications précédentes, dans lequel le au moins un autre extrait est un extrait végétal contenant des vésicules.

14. Solution aqueuse d'un extrait végétal, qui comporte au moins un acide triterpénique et/ou un dérivé d'un acide triterpénique, la solution ayant, à un pH de 5 à 9 en la présence d'un additif stabilisant la solution physiologique aqueuse d'acide triterpénique, une teneur en acide triterpénique de plus de 1 µg/ml, l'additif stabilisant étant un polysaccharide ou une cyclodestrine, et dans laquelle le dérivé d'un acide triterpénique comprend de l'acide asiatique, de l'acide bétulique, de l'acide boswellique, de l'acide corrosolique, de l'acide glycyrrhétinique, de l'acide madécassique, de l'acide oléanolique, de l'acide phellonique et/ou de l'acide ursolique.

15. Solution suivant la revendication 14, dans laquelle l'extrait végétal est un extrait de gui, de bouleau, de platane et/ou d'autres plantes contenant un triterpénoïde ou de leurs parties.

16. Solution suivant l'une des revendications 17 à 19, dans laquelle la concentration en sel de la solution est comprise entre 5 et 140 mM.

17. Solution suivant la revendication 16, dont la concentration en sel est comprise entre 25 mM et 35 mM.

18. Solution suivant l'une des revendications 14 à 17, qui contient un extrait de gui contenant des vésicules.

19. Utilisation d'une solution aqueuse suivant l'une des revendications 14 à 17 pour la préparation d'un agent thérapeutique.

20. Utilisation d'une solution aqueuse suivant l'une des revendications 14 à 17 pour la préparation d'un complément alimentaire.

21. Utilisation d'une solution aqueuse suivant l'une des revendications 14 à 17 pour la préparation d'une préparation injectable physiologiquement.

22. Utilisation d'une solution aqueuse suivant l'une des revendications 14 à 17 pour la préparation d'une préparation injectable physiologiquement pour la thérapie tumorale.

23. Utilisation d'une solution aqueuse suivant l'une des revendications 14 à 17 pour la préparation d'une préparation injectable physiologiquement pour la thérapie de l'hépatite.

24. Utilisation d'une solution aqueuse suivant l'une des revendications 14 à 17 pour la préparation d'un agent thérapeutique de traitement thérapeutique de maladies tumorales de la peau.

25. Utilisation d'une solution aqueuse suivant l'une des revendications 14 à 17 pour la préparation d'un agent thérapeutique pour lutter contre les virus et les bactéries.
